# FASCICULE DE BREVET EUROPEEN

(11) **EP 4 477 165 B1**
(45) Date de publication et mention de la délivrance du brevet: **22.10.2025**
(21) Numéro de dépôt: 24182324.4
(22) Date de dépôt: 14.06.2024
(51) Int. Cl.: A61B 17/70, A61B 17/80

(54) **SYSTÈME DE FIXATION SACRÉE ET ENSEMBLE COMPRENANT UN TEL SYSTÈME**
SYSTEM ZUR BEFESTIGUNG EINES SACKES UND ANORDNUNG MIT EINEM SOLCHEN SYSTEM
SACRAL ATTACHMENT SYSTEM AND ASSEMBLY COMPRISING SUCH A SYSTEM

(30) Priorité: 16.06.2023 FR 2306212
(43) Date de publication de la demande: 18.12.2024
(73) Titulaire: S.M.A.I.O, 69800 Saint-Priest (FR)
(72) Inventeur: GEHRCHEN, Poul, Martin, 2900 HELLERUP (DK); SILVESTRE, Clément, 69340 FRANCHEVILLE (FR); ROUSSOULY, Pierre, 69450 SAINT CYR AU MONT D'OR (FR); CHAROSKY, Sébastien, 31320 VIEILLE TOULOUSE (FR); ROUSSOULY, Jean-Charles, 69250 POLEYMIEUX (FR)
(74) Mandataire: Lavoix

(56) Documents cités:
- EP-A1- 1 915 958
- WO-A1-95/23559
- US-A1- 2012 022 595
- US-A1- 2013 085 534

## Description

La présente invention concerne un système de fixation sacrée et un ensemble comprenant un tel système de fixation.

Pour les montages d'arthrodèse postérieure du rachis, il est connu d'utiliser un système de fixation sacrée, par exemple sous forme de plaque venant se fixer au sacrum d'un patient, plus particulièrement aux vertèbres S1 et S2, à l'aide de vis.

Le système de fixation sacrée sous forme de plaque permet d'une part un décalage distal de la connexion entre la tige vertébrale du montage d'arthrodèse et le système de fixation sacrée, comparativement à l'utilisation d'une seule vis pédiculaire dans la vertèbre S1. Cela permet de concentrer la lordose lombaire proche du sacrum sans risquer de conflit entre des ancrages de la vertèbre L5 et du sacrum.

D'autre part, lorsque les montages d'arthrodèse postérieure du rachis atteignent le pelvis, le système de fixation sacrée représente le socle du montage. Par conséquent, le système de fixation sacrée requiert une robustesse lui assurant notamment une résistance élevée à une force d'arrachement auquel il est soumis lorsqu'il est assemblé sur le sacrum. Le système de fixation sacrée sous forme de plaque, bénéficiant de deux vis insérées respectivement dans les vertèbres S1 et S2 du sacrum, voit sa robustesse améliorée comparativement à une vis pédiculaire insérée dans le sacrum.

Ces systèmes donnent globalement satisfaction, mais la tenue des vis de fixation du montage peut être insuffisante dans le temps, ce qui fragilise le montage et limite sa durée de vie.

US 2013/085534 A1 décrit un connecteur pour ancrages osseux comprenant, un premier composant engageable avec un ancrage osseux primaire et ajustable en rotation, un second composant formant une douille pour recevoir un ancrage osseux secondaire dans diverses orientations angulaires et un mécanisme de fermeture pour fixer l'ancrage osseux secondaire dans une orientation choisie.

Le but de la présente invention est de proposer un système de fixation sacrée amélioré, présentant une robustesse et une durée de vie améliorées.

A cet effet, l'invention a pour objet un système de fixation sacrée, comprenant une plaque comprenant une face proximale et une face distale, opposée à la face proximale, un plan de plaque s'étendant entre les faces distale et proximale, un premier logement s'étendant selon un premier axe en reliant entre elles les faces distale et proximale, le premier logement incluant à la fois un premier trou, qui débouche sur la face distale, et un premier fond, dans lequel est percé le premier trou et qui relie le premier trou et la face proximale, un deuxième logement s'étendant selon un deuxième axe en reliant entre elles les faces distale et proximale, le deuxième logement incluant à la fois un deuxième trou, qui débouche sur la face distale, une surface taraudée, qui débouche sur la face proximale, et un deuxième fond, qui est de forme concave, dans lequel est percé le deuxième trou et qui relie le deuxième trou et la surface taraudée, et un axe de plaque appartenant au plan de plaque et sécant avec les premier et deuxième axes, le deuxième axe formant, en projection dans un plan perpendiculaire à l'axe de plaque, un premier angle compris entre 30 et 60 degrés avec le plan de plaque, et le deuxième axe et l'axe de plaque formant entre eux un deuxième angle compris entre 45 degrés et 110 degrés. Le système comprend également une première vis, qui, lorsque le système est dans une configuration assemblée, est reçue dans le premier logement, en s'étendant dans le premier trou et en émergeant de la face distale de manière à pouvoir pénétrer dans la vertèbre S1 du sacrum d'un patient et une deuxième vis, qui comprend une tige filetée et une tête, la tête étant sensiblement sphérique de manière complémentaire au deuxième fond, la deuxième vis étant, lorsque le système est dans la configuration assemblée, reçue dans le deuxième logement de sorte que la tête est en appui sur le deuxième fond et la tige s'étend dans le deuxième trou et émerge de la face distale de manière à pouvoir pénétrer au moins dans la vertèbre S2 du sacrum du patient. Le système comprend également un bouchon de verrouillage, qui comprend une jupe dont une face extérieure est filetée de manière complémentaire à la surface taraudée et dont une face intérieure est complémentaire de la tête de la deuxième vis, et qui, lorsque le système est dans la configuration assemblée, est reçu dans le deuxième logement en interposant la jupe radialement entre la tête de la deuxième vis et la surface taraudée, de manière que la tête de la deuxième vis est reçue dans la jupe et est appuyée contre la face intérieure et un dispositif de connexion, qui, lorsque le système est dans la configuration assemblée, est disposé sur la face proximale entre les premier et deuxième logements et relie la plaque à une tige vertébrale.

Une des idées à la base de l'invention est de faire prendre à l'angulation de l'axe du deuxième logement, qui est fixe pour une plaque donnée, une valeur dans une large plage de valeurs prédéterminées, à savoir la plage s'étendant de 45 degrés à 110 degrés. Ainsi, le système de fixation autorise de choisir la position la plus adaptée pour la deuxième vis lorsqu'elle est en configuration assemblée, afin de garantir qu'elle pénètre dans la vertèbre S2 du sacrum du patient sans endommager d'autres organes, comme des nerfs ou des vaisseaux sanguins.

De plus, lorsque le système de fixation est en configuration assemblée et implanté dans le patient, il est soumis à une force d'arrachement qui est susceptible de causer son arrachement du sacrum, phénomène qu'il convient de limiter. L'invention permet, grâce à l'angulation du deuxième logement, d'obtenir une meilleure tenue mécanique du système de fixation. En effet, la deuxième vis pénètre, selon l'angulation imposée par le deuxième logement, au moins dans la vertèbre S2 du patient, et peut avantageusement s'étendre au-delà si nécessaire. Ainsi, l'invention permet d'adapter l'orientation du deuxième logement, et donc de la deuxième vis, au contexte particulier à chaque patient pour optimiser la tenue mécanique de la deuxième vis dans chaque cas et éviter d'endommager d'autres organes.

La robustesse du système de fixation sacrée est assurée dans le temps également grâce au bouchon de verrouillage. La jupe du bouchon de verrouillage est interposée radialement entre la surface taraudée du deuxième logement et la tête de la deuxième vis, et appuie contre la tête de la deuxième vis, ce qui entraîne la mise en appui de la tête de la deuxième vis contre le fond du deuxième logement. Ces deux appuis génèrent une force de friction suffisante pour bloquer totalement la deuxième vis. En particulier, la deuxième vis ne peut pas pivoter dans le deuxième logement. Elle ne peut donc ainsi ni se dévisser et s'extraire de la plaque, ni pivoter dans son logement et fragiliser le système, en modifiant l'orientation relative de la plaque et de la deuxième vis. Le bouchon de verrouillage empêche ainsi la deuxième vis de bouger lorsque que le système est en configuration assemblée. L'invention est donc robuste et a une résistance, notamment à la force d'arrachement, optimisée, ce qui permet une bonne tenue mécanique dans l'os et donc une durée de vie importante.

Le système peut comprendre l'une ou plusieurs des caractéristiques suivantes, prises isolément ou suivant toute combinaison techniquement possible :
- Le deuxième angle est compris entre 70 degrés et 110 degrés, de préférence compris entre 85 et 95 degrés, et la deuxième vis est dimensionnée pour, dans la configuration assemblée, traverser la vertèbre S2 et atteindre l'os iliaque du patient.
- La plaque comprend en outre une dépression et le dispositif de connexion comprend :
   - une extension, filetée, liée fixement à la dépression de la plaque, l'extension s'étendant selon un axe d'extension ;
   - un connecteur, comprenant une calotte proximale et une calotte distale, la calotte distale étant complémentaire de la dépression, chaque calotte comprenant :
      ∘ un orifice, de diamètre supérieur à un diamètre de l'extension, les orifices respectifs des calottes proximale et distale étant coaxiaux et centrés sur un axe de connecteur,
      ∘ une surface d'appui, les surfaces d'appui étant en regard l'une de l'autre selon l'axe de connecteur ;
      chaque calotte étant déplaçable selon l'axe de connecteur afin de mettre les surfaces d'appui en contact l'une avec l'autre selon l'axe de connecteur et de serrer le connecteur autour de la tige vertébrale,
   - un écrou, comprenant une partie de serrage, la partie de serrage comprenant une surface distale complémentaire à la calotte proximale,
   et dans lequel, lorsque le système est en configuration assemblée :
   - le connecteur est enfilé sur l'extension par les orifices des calottes distale et proximale ; et
   - l'écrou est vissé sur l'extension, la surface distale est en appui contre la calotte proximale, l'écrou maintient la calotte distale en appui sur la dépression et maintient les surfaces d'appui en contact selon l'axe de connecteur ; et
   - le connecteur est serré autour de la tige vertébrale.
- Les calottes proximale et distale sont hémisphériques et lorsque le système est en configuration assemblée, les calottes inférieure et supérieure sont co-radiales.
- L'écrou comprend en outre une partie de vissage, portée par la partie de serrage, configurée pour se détacher de la partie de serrage lorsqu'un couple égal à un couple maximum de vissage est appliqué à la partie de vissage.
- L'extension comprend une ligne de moindre résistance la rendant sécable lorsqu'un couple en flexion ou en torsion égal à un couple de rupture en flexion ou en torsion est appliqué à l'extension.
- Le dispositif de connexion comprend :
   - un pion, porté fixement par la plaque ;
   - une pièce en tulipe s'étendant selon un axe de pièce en tulipe, le pion et la pièce en tulipe formant entre elles une liaison rotule, la pièce en tulipe comprenant une ouverture traversante taraudée ;
   - un palet, comprenant une surface distale complémentaire au pion et une surface proximale complémentaire à la tige vertébrale ;
   - un bouchon, adapté à être vissé dans l'ouverture de la pièce en tulipe,
   lorsque le système est dans la configuration assemblée :
   - le palet est disposé dans l'ouverture de la pièce en tulipe, la surface distale du palet étant en appui contre le pion ;
   - la tige vertébrale traverse l'ouverture de la pièce en tulipe, en appui contre la surface proximale du palet ;
le bouchon est vissé dans l'ouverture de la pièce en tulipe et maintient la tige vertébrale, le palet et le pion en appui les uns contre les autres.
- L'axe de plaque passe par le pion.
- Le pion est décalé par rapport à l'axe de plaque dans une direction perpendiculaire à l'axe de plaque.

L'invention a également pour objet un ensemble de fixation sacré comprenant le système décrit ci-dessus et un guide de perçage comprenant :
- une surface distale morpho-adaptée à une région postérieure du sacrum du patient ;
- un premier trou de guide centré selon un premier axe de guide, et
un deuxième trou de guide centré selon un deuxième axe de guide, le premier trou de guide et le deuxième trou de guide étant orientés de manière identique à la première et à la deuxième vis lorsque le système est en configuration assemblée.

L'invention apparaîtra plus clairement à la lecture de la description qui va suivre, donnée uniquement à titre d'exemple non limitatif et faite en se référant aux figures dans lesquelles :
- [Fig. 1] la figure 1 est une vue d'un système de fixation sacrée implanté sur un patient selon un premier mode de réalisation de l'invention ;
- [Fig. 2] la figure 2 est une vue en perspective d'une plaque du système de fixation sacrée selon le premier mode de réalisation ;
- [Fig. 3] la figure 3 représente, sur deux inserts a) et b), des vues en élévation de la plaque de la figure 2, associée à des première et deuxième vis de fixation, l'insert a) étant une vue en élévation selon la flèche Illa sur la figure 2 et l'insert b) étant une vue en élévation selon la flèche IIIb sur l'insert a) ;
- [Fig. 4] la figure 4 est une coupe selon la ligne IV-IV de la figure 1 ;
- [Fig. 5] la figure 5 montre, sur deux inserts a) et b), des vues en élévation de la plaque associée aux première et deuxième vis de fixation de la figure 3, la plaque et les vis de fixation étant schématiquement associées à de la matière osseuse, l'insert a) étant une vue correspondant à l'insert a) de la figure 3, et l'insert b) étant une vue en élévation selon la flèche Vb sur l'insert a) ;
- [Fig. 6] la figure 6 est une coupe schématique selon le plan VI-VI de l'insert b) de la figure 3 ;
- [Fig. 7] la figure 7 représente, sur deux inserts a) et b), des sections schématiques selon la ligne VII-VII de l'insert b) de la figure 3, où la plaque est associée à un dispositif de connexion, l'insert a) étant une section où le dispositif de connexion est en configuration libre et l'insert b) étant une section où le dispositif de connexion est en configuration serrée;
- [Fig. 8] la figure 8 représente, sur deux inserts a) et b), des sections schématiques où le débattement du dispositif de connexion est représenté, l'insert a) étant une section correspondant à l'insert a) de la figure 7 et l'insert b) étant une section schématique selon le plan VI-VI de l'insert b) de la figure 3, la plaque étant associée au dispositif de connexion ;
- [Fig. 9] la figure 9 est une vue similaire à la figure 4, selon un deuxième mode de réalisation de l'invention;
- [Fig. 10] la figure 10 montre deux inserts a) et b), respectivement similaires aux inserts a) et b) de la figure 5, la plaque étant selon le deuxième mode de réalisation de l'invention ;
- [Fig. 11] la figure 11 est une vue en perspective d'une tige vertébrale d'une plaque et d'un connecteur selon un troisième mode de réalisation de l'invention ;
- [Fig. 12] la figure 12 est une coupe selon le plan XII-XII de la plaque de la figure 11, où le débattement du dispositif de connexion est représenté ;
- [Fig. 13] la figure 13 est une vue similaire à la figure 11, la plaque étant selon un quatrième mode de réalisation de l'invention ; et
- [Fig. 14] la figure 14 représente sur un insert a) une vue en élévation d'un guide de perçage et sur un insert b) une vue en élévation du guide de perçage selon la flèche XIVb de l'insert a).

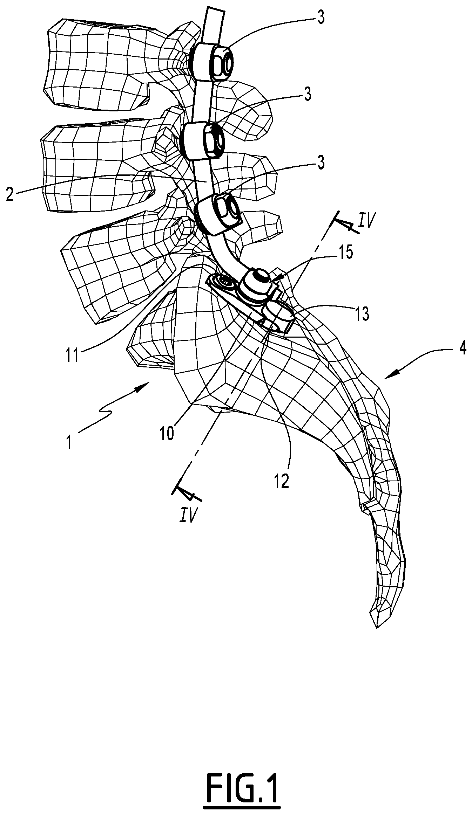

Sur la figure 1 est représenté un système de fixation sacrée 1, aussi appelé système 1. Le système 1 appartient à un montage d'arthrodèse, comprenant également une tige vertébrale 2 et plusieurs vis pédiculaires 3. Le système 1 est représenté ici dans une configuration assemblée et fixé sur le sacrum 4 d'un patient, plus particulièrement aux vertèbres S1 et S2 du sacrum 4 du patient, comme expliqué plus en détail par la suite.

Le montage d'arthrodèse concerne ici un des deux côtés parmi un côté gauche et un côté droit de la colonne vertébrale du patient. Sur la figure 1, le montage d'arthrodèse représenté est le côté gauche. Le cas échéant, un second montage est fixé sur le côté droit, qui comprend des aménagements globalement symétriques à ceux du montage d'arthrodèse du côté gauche par rapport à un plan sagittal du patient. En particulier, le système du montage d'arthrodèse situé sur le côté droit est globalement symétrique au système 1 par rapport au plan sagittal du patient.

Le système 1 comprend une plaque 10, une première vis de fixation 11, une deuxième vis de fixation 12, un bouchon de verrouillage 13 et un dispositif de connexion 15.

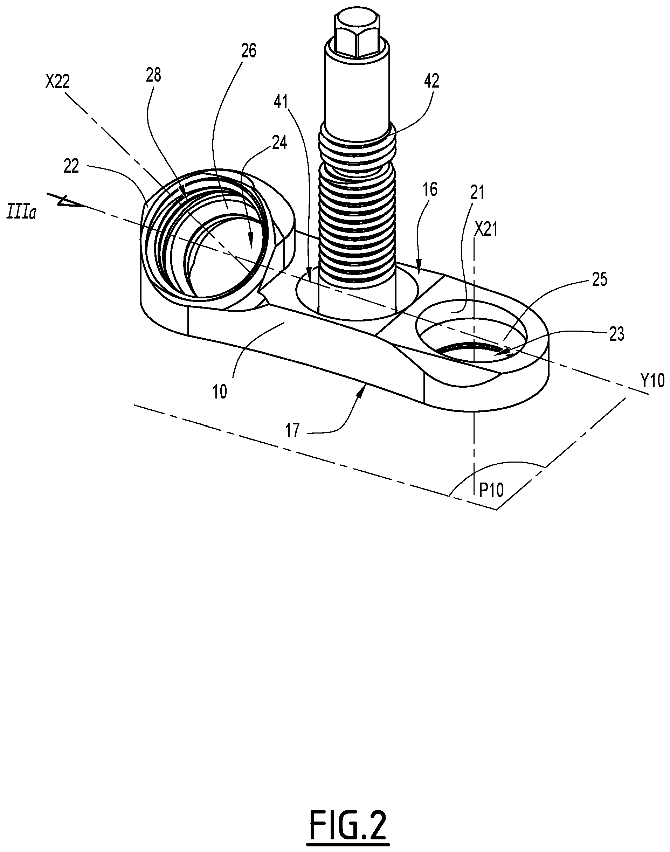

Comme bien visible sur la figure 2, la plaque 10 comprend une face proximale 16 et une face distale 17, opposée à la face proximale 16, dans le sens où les faces proximale 16 et distale 17 sont séparées l'une de l'autre par l'épaisseur de la plaque 10, c'est-à-dire la plus petite des trois dimensions de la plaque 10. On note P10 un plan de plaque géométrique qui s'étend entre les faces proximale 16 et distale 17.

Dans le mode de réalisation illustré aux figures 1 à 8, la face distale 17 est plate, c'est-à-dire parallèle au plan P10. De manière alternative non représentée, la face distale est courbée, en particulier concave, notamment afin de mieux épouser une courbure cyphotique du sacrum 4.

La plaque 10 comprend deux logements distincts, à savoir un premier logement 21 et un deuxième logement 22. Le premier logement 21 relie l'une à l'autre les faces proximale 16 et distale 17, en s'étendant selon un premier axe X21. Le deuxième logement 22 relie l'une à l'autre les faces proximale 16 et distale 17 en s'étendant selon un deuxième axe X22. Les premier et deuxième axes X21 et X22 définissent un axe de plaque Y10, par leur intersection avec le plan de plaque P10. Autrement dit, l'axe de plaque Y10 appartient au plan de plaque P10 et est sécant avec les premier et deuxième axes X21 et X22.

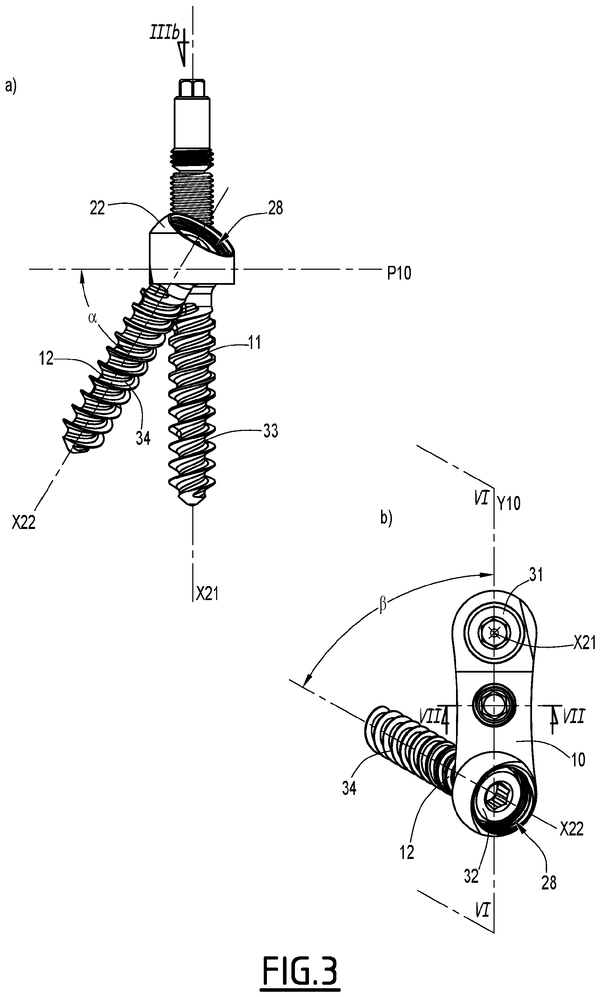

Comme bien visible sur la figure 2 et sur les inserts a) et b) de la figure 3, le premier axe X21 est ici perpendiculaire au plan de plaque P10.

Egalement comme bien visible sur la figure 2 et sur les inserts a) et b) de la figure 3, le deuxième axe X22 est lui aussi transversal au plan de plaque P10, sans toutefois être parallèle au premier axe X21.

Plus précisément, comme montré sur l'insert a) de la figure 3, le deuxième axe X22 forme, en projection dans un plan géométrique perpendiculaire à l'axe de plaque Y10, un premier angle α avec le plan de plaque P10, ce premier angle α étant compris entre 30 et 60 degrés, préférentiellement égal à 45 degrés, le premier angle α étant mesuré sur le côté de la plaque 10, destiné à être tourné à l'opposé du plan sagittal du patient.

De plus, comme montré sur l'insert b) de la figure 3, le deuxième axe X22 forme un deuxième angle β avec l'axe de plaque Y10, ce deuxième angle β étant compris entre 70 et 110 degrés dans la forme de réalisation considérée sur les figures 1 à 8, le deuxième angle β étant mesuré sur le côté de la plaque 10 destiné à être tourné vers le haut par rapport au patient. Autrement dit, le deuxième angle β est formé dans un plan géométrique formé par l'axe de plaque Y10 et le deuxième axe X22. Pour des raisons qui apparaîtront plus loin, le deuxième angle β est préférentiellement compris entre 85 et 95 degrés, voire égal à 90 degrés.

Comme bien visible sur la figure 2, le premier logement 21 comprend un premier trou 23, qui débouche sur la face distale 17, et un premier fond 25 qui relie le premier trou 23 et la face proximale 16. Le premier trou 23 est percé dans le premier fond 25, en étant ici centré sur le premier axe X21. Le premier trou 23 est avantageusement tronconique, de telle sorte qu'un diamètre du premier trou 23 mesuré à la jonction entre le premier trou 23 et la face distale 17 est supérieur à un diamètre mesuré à la jonction entre le premier trou 23 et le premier fond 25. Le premier fond 25 est ici de forme sensiblement hémisphérique, c'est-à-dire en forme d'une portion de sphère, centrée sur le premier axe X21.

Le deuxième logement 22 comprend à la fois un deuxième trou 24, qui débouche sur la face distale 17, une surface taraudée 28, qui débouche sur la face proximale 16, et un deuxième fond 26 qui relie l'un à l'autre le deuxième trou 24 et la surface taraudée 28. Le deuxième trou 24 est percé dans le deuxième fond 26 et est ici centré sur le deuxième axe X22. Le deuxième trou 24 est avantageusement tronconique, de telle sorte qu'un diamètre du deuxième trou 24 mesuré à la jonction entre le deuxième trou 24 et la face distale 17 est supérieur à un diamètre mesuré à la jonction entre le deuxième trou 24 et le deuxième fond 26. Le deuxième fond 26 est sensiblement de forme concave, en particulier hémisphérique, c'est-à-dire qu'il est de la forme d'une portion de sphère, centrée sur le deuxième axe X22. Le deuxième fond 26 a avantageusement un diamètre égal à un diamètre du premier fond 25. La surface taraudée 28 est centrée sur le deuxième axe X22 et s'étend entre le deuxième fond 26 et la face proximale 16.

Pour des raisons qui apparaîtront plus loin, la plaque 10 comprend une dépression 30, située plus précisément sur la face proximale 16. La dépression 30 est avantageusement hémisphérique et centrée sur un axe de dépression X30, perpendiculaire au plan de plaque P10. L'axe de dépression X30 est situé entre les premier et deuxième logements 11 et 12, et, avantageusement, l'axe de dépression X30 intersecte l'axe de plaque Y10.

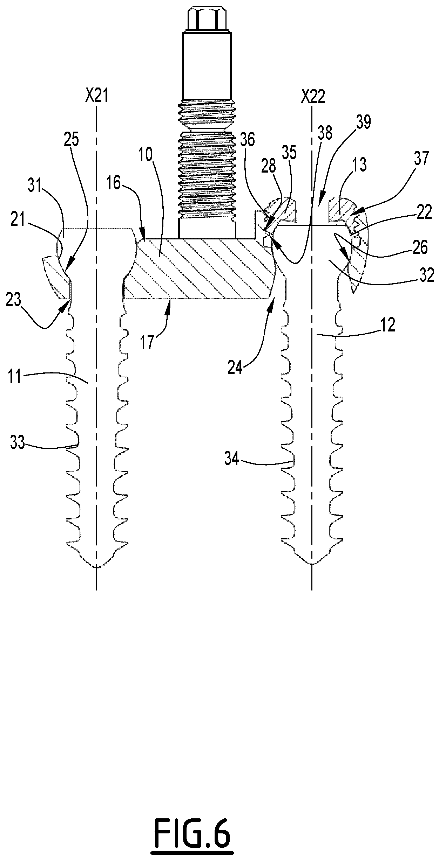

Comme bien visible sur la figure 6, la première vis 11 comprend une tête 31 sensiblement sphérique, de forme complémentaire au premier fond 25, et une tige 33 filetée. La deuxième vis 12 comprend également une tête 32 sensiblement sphérique, de forme complémentaire au deuxième fond 26, et une tige 34 filetée. La première et la deuxième vis 11 et 12 sont avantageusement identiques l'une à l'autre.

Lorsque le système 1 est en configuration assemblée, comme représenté sur les figures 3 à 6, la première vis 11 est reçue dans le premier logement 21. La tête 31 de la première vis 11 est en appui contre le premier fond 25, et la tige 33 s'étend dans le premier trou 23 et le traverse, en émergeant ainsi en saillie de la face distale 17 pour pénétrer dans la vertèbre S1 du sacrum 4 et ainsi fixer la plaque 10 au sacrum 4 du patient. Comme mentionné précédemment, la tête 31 de la première vis 11 et le premier fond 25 ont une forme complémentaire de sorte que la tête 31 et le premier fond 25 sont appuyés l'un contre l'autre en formant un contact sphère-sphère. Comme illustré schématiquement sur les figures 3, 5 et 6, dans la configuration assemblée, la première vis 11 s'étend en longueur sensiblement selon le premier axe X21, c'est-à-dire que son axe longitudinal central se retrouve confondu avec le premier axe X21, ou bien forme avec ce dernier un angle de quelques degrés seulement, typiquement de moins de 15 degrés.

Selon une forme de réalisation alternative et non représentée, la première vis 11 forme un angle supérieur à 15 degrés avec le premier axe X21.

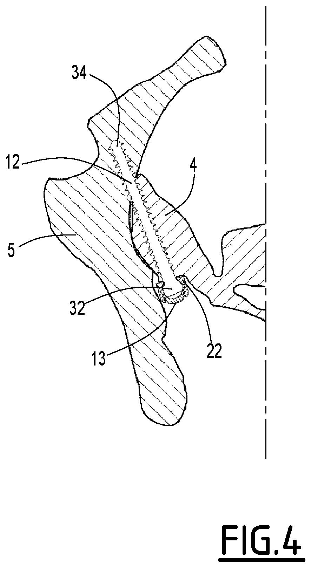

Lorsque le système 1 est en configuration assemblée, comme représenté sur les figures 3 à 6, la deuxième vis 12 est reçue dans le deuxième logement 22. La tête 32 de la deuxième vis 12 est en appui contre le deuxième fond 26, selon un contact sphère-sphère, et la tige 34 s'étend dans le deuxième trou 24 et le traverse, émergeant ainsi en saillie de la face distale 17 pour pénétrer dans la vertèbre S2 du sacrum 4 du patient, comme visible figure 4 et ainsi fixer la plaque 10 au sacrum 4 du patient, en complément de la première vis 11. Comme mentionné précédemment, et de manière similaire à la première vis 11, la tête 32 et le deuxième fond 26 ont une forme complémentaire, de sorte que la tête 32 de la deuxième vis 12 et le deuxième fond 26 sont appuyés l'un contre l'autre en formant un contact sphère-sphère.

En pratique, dans la configuration assemblée, la deuxième vis 12 s'étend en longueur sensiblement selon le deuxième axe X22, c'est-à-dire que son axe longitudinal central se retrouve confondu avec le deuxième axe X22, comme illustré schématiquement sur les figures 3, 5 et 6, ou bien forme avec ce dernier un angle de quelques degrés seulement, typiquement de moins de 15 degrés. Dans tous les cas, l'angulation du deuxième axe X22, telle que définie plus haut au travers des premier et deuxième angles α et β, permet que, en configuration assemblée, la deuxième vis 12 pénètre la vertèbre S2 jusqu'à pouvoir, dès lors que la deuxième vis 12 est dimensionnée en longueur de manière ad hoc, traverser de part en part la vertèbre S2 et pénétrer dans l'os iliaque 5 du patient, comme illustré schématiquement sur la figure 4. A titre d'exemple préférentiel, la deuxième vis 12 présente à cet effet un dimensionnement longitudinal compris entre 40 mm et 120 mm, préférentiellement entre 50 mm et 110 mm. Cette fixation à la fois dans la vertèbre S2 du sacrum 4 et dans l'os iliaque 5 du patient optimise une tenue mécanique du système 1.

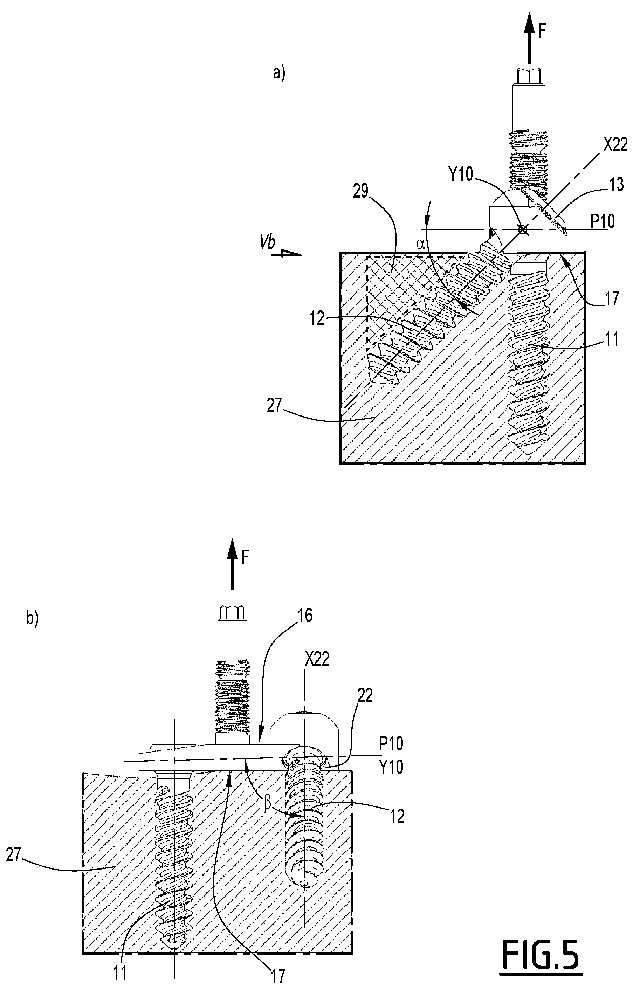

On notera que, en service, c'est-à-dire lorsque le patient sur qui le système 1 en configuration assemblée a été implanté, sollicite sa colonne vertébrale en mouvement, le système 1 est typiquement soumis à une force d'arrachement F qui, comme illustré sur les inserts a) et b) de la figure 5, s'exerce perpendiculairement au plan de plaque P10, en étant orienté de la face distale 17 vers la face proximale 16. La deuxième vis 12 s'oppose efficacement à cette force F de par son alignement sensiblement avec le deuxième axe X22.

Pour mieux illustrer ce phénomène, on a représenté sur les inserts a) et b) de la figure 5 les première et deuxième vis 11 et 12 pénétrant dans de la matière osseuse 27, la matière osseuse 27 étant représentée schématiquement par une zone hachurée représentant de manière indifférenciée le sacrum 4 et l'os iliaque 5 du patient. L'os 27 comprend une région osseuse 29, représentée en pointillés et doublement hachurée. Cette région osseuse 29 est localisée entre la deuxième vis 12 et le plan de plaque P10 et limite de mouvement de la deuxième vis 12 dû à la force d'arrachement F, ce qui empêche au système 1 d'être arraché. L'existence de la région osseuse 29 tient à l'angulation de X22, et notamment de la valeur du premier angle α. Il est donc particulièrement avantageux d'avoir le premier angle α compris entre 30 et 60 degrés pour maximiser un volume de la région osseuse 29.

Le bouchon de verrouillage 13 permet d'empêcher la deuxième vis S2 de bouger par rapport à la plaque 10, notamment de se dévisser, sous l'action de la force F et/ou d'autres forces. Comme bien visible sur les figures 4 et 6, le bouchon de verrouillage 13 comprend à cet effet une jupe 36 centrée sur un axe géométrique qui, dans la configuration assemblée du système 1, est alignée sur le deuxième axe X22. Par définition, la jupe 36 comprend deux faces séparées l'une de l'autre par son épaisseur, à savoir une face extérieure 37 et une face intérieure 38. La face extérieure 37 est filetée de manière complémentaire à la surface taraudée 28, permettant ainsi au bouchon de verrouillage 13 d'être logé et fixé à l'intérieur du deuxième logement 22 par vissage entre la surface taraudée 28 et le filetage de la face extérieure 37 lorsque le système 1 est en configuration assemblée. La face intérieure 38 est complémentaire de la tête 32 de la deuxième vis 12, en étant ainsi hémisphérique, permettant donc à la tête 32 d'être reçue à l'intérieur de la jupe 36 et d'être appuyée, de manière complémentaire, contre la face intérieure 38.

Dans la configuration assemblée du système 1, la jupe 36 se retrouve interposée, radialement au deuxième axe X22, entre la tête 32 de la deuxième vis 12 et la surface taraudée 28 du deuxième logement 22. Le bouchon de verrouillage 13 étant vissé dans le deuxième logement 22, il appuie sur la tête 32 de la deuxième vis 12, afin de mettre en appui la face intérieure 38 de la jupe 36 sur la tête 32, et ainsi plaquer la tête 32 contre le deuxième fond 26. Ainsi, la deuxième vis 12 est maintenue immobile dans le deuxième logement 22, y compris en pivotement, en particulier autour du deuxième axe X22.

A l'extrémité axiale de la jupe 36, qui est tournée axialement à l'opposé de la deuxième vis 12 dans la configuration assemblée du système 1, le bouchon de verrouillage 13 comporte avantageusement une tête 35. Une empreinte 39 est réalisée dans une partie centrale de la tête 35, l'empreinte 39 permettant le vissage du bouchon de verrouillage 13, par exemple par une clé de vissage. La jupe 36 est portée fixement par la tête 35, en s'étendant depuis une partie périphérique de la tête 35.

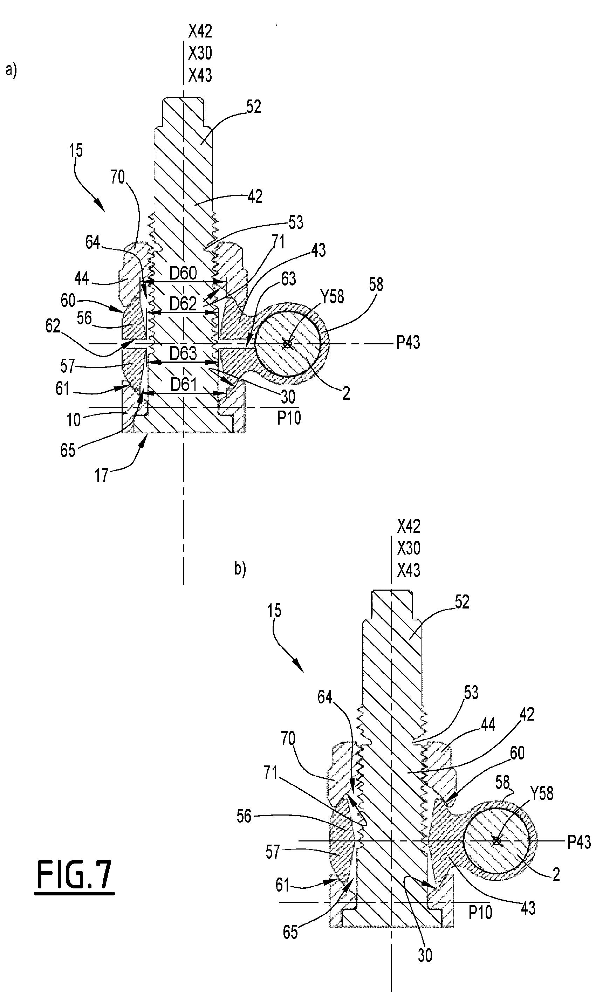

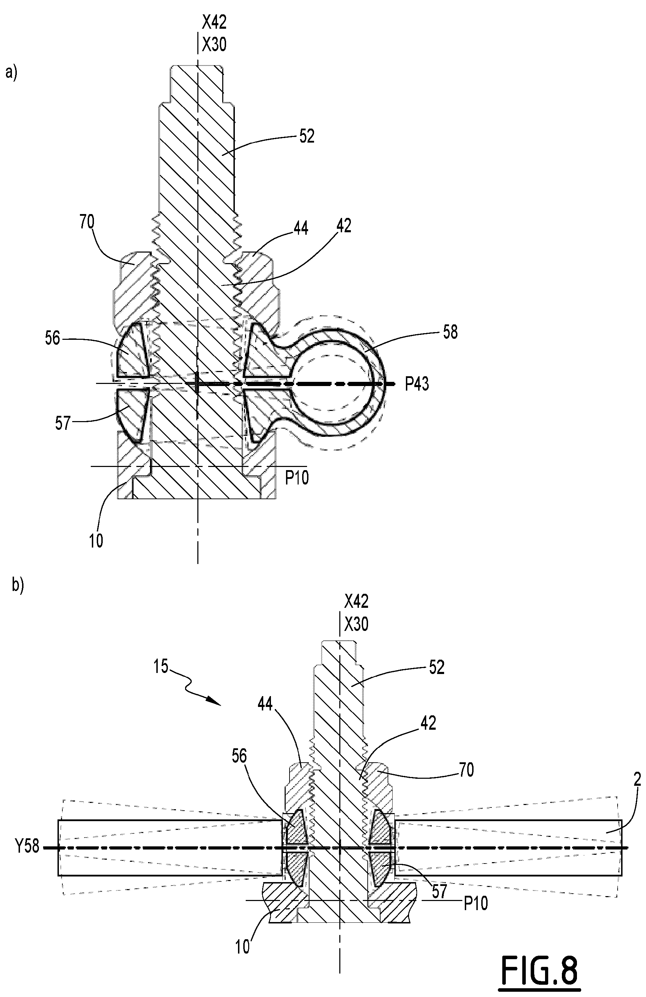

Comme bien visible sur les inserts a) et b) des figures 7 et 8, le dispositif de connexion 15 comprend une extension 42, un connecteur 43 et un écrou 44.

L'extension 42 s'étend selon un axe d'extension X42, en étant fixée à la plaque 10 au niveau de la dépression 30 de telle sorte que les axes d'extension X42 et de dépression X30 sont confondus. Comme représenté sur les inserts a) et b) des figures 7 et 8, l'extension 42 est formée par une tige de vis traversant la plaque 10 selon l'axe de dépression X30, la tige de vis appartenant à une vis qui est soudée à la plaque 10. L'extension 42 est au moins partiellement filetée entre ses extrémités proximale et distale, une partie terminale proximale 52 de l'extension 42 n'étant ici avantageusement pas filetée.

L'extension 42 présente avantageusement une ligne de moindre résistance 53, conçue pour se rompre lorsqu'un couple en flexion ou en torsion égal à un couple de rupture en flexion ou en torsion est appliqué sur l'extrémité proximale de l'extension 42. Cela permet notamment de limiter l'encombrement de l'extension 42 une fois que le système 1 a été assemblé.

Le connecteur 43 s'étend selon un axe de connecteur X43 et comprend une calotte proximale 56, une calotte distale 57 et une bague 58. Les calottes proximale 56 et distale 57 sont situées de part et d'autre d'un plan médian P43 et sont centrées sur l'axe de connecteur X43.

La calotte proximale 56 comprend une surface proximale 60 qui est de forme hémisphérique. La calotte proximale 56 comprend également une surface d'appui 62, qui est une surface plane. La surface d'appui 62 est disposée entre la surface proximale 60 et la calotte distale 57 selon l'axe de connecteur X43.

La calotte proximale 56 comprend aussi un orifice 64. L'orifice 64 traverse la calotte proximale 56 selon l'axe de connecteur X43 et relie la surface proximale 60 de la calotte proximale 56 à la surface d'appui 62. Un diamètre de l'orifice 64 est supérieur ou égal au diamètre de l'extension 42. Avantageusement, et ainsi que représenté sur les inserts des figures 7 et 8, l'orifice 64 est tronconique, de sorte qu'un diamètre D60, mesuré à la jonction entre l'orifice 64 et la surface proximale 60 de la calotte proximale 56 est supérieur à un diamètre D62, mesuré à la jonction entre l'orifice 64 et la surface d'appui 62.

La calotte distale 57 comprend une surface distale 61. La surface distale 61 de la calotte distale 57 est elle aussi de forme hémisphérique, centrée sur l'axe de connecteur X43 et de forme complémentaire à la dépression 30. La calotte distale 57 comprend aussi une surface d'appui 63. La surface d'appui 63 est parallèle à et en regard de la surface d'appui 62 de la calotte proximale 56 selon l'axe de connecteur X43. La surface d'appui 63 est située entre la surface d'appui 62 et la surface distale 61 selon l'axe de connecteur X43.

La calotte distale 57 comprend également un orifice 65. L'orifice 65 traverse la calotte distale 57 selon l'axe de connecteur X43 et relie la surface distale 61 de la calotte distale 57 à la surface d'appui 63. Un diamètre de l'orifice 65 est supérieur ou égal à un diamètre de l'extension 42. Avantageusement, et ainsi que représenté sur les inserts des figures 7 et 8, l'orifice 65 est tronconique, de sorte qu'un diamètre D61, mesuré à la jonction entre l'orifice 65 et la surface distale 61 de la calotte distale 57 est supérieur à un diamètre D63, mesuré à la jonction entre l'orifice 65 et la surface d'appui 63.

De manière avantageuse, et ainsi que représenté figures 7 et 8, les calottes proximale 56 et distale 57 sont symétriques par rapport au plan médian P43, en particulier, les surfaces proximale 60 et distale 61 ont un rayon de courbure identique.

La bague 58 a une forme tubulaire, s'étendant selon un axe de bague Y58, orthogonal à l'axe de connecteur X43 et appartenant au plan médian P43. La bague 58 est donc symétrique par rapport au plan médian P43. La bague 58 est reliée fixement aux calottes proximale 56 et distale 57, et est adaptée pour entourer la tige vertébrale 2.

Lorsque le connecteur 43 est enfilé sur l'extension 42, la surface proximale 61 de la calotte distale 57 épouse la dépression 30 selon un contact sphère-sphère. Le contact sphère-sphère entre la calotte distale 57 et la dépression 30, ainsi que les orifices 64 et 65, tronconiques, permet au connecteur 43 de se débattre autour de l'extension 42 et prendre plusieurs orientations par rapport à la plaque 10. Par exemple, l'axe de connecteur 43 peut faire un angle avec l'axe d'extension X42 compris entre 0 et 15 degrés, l'axe de bague Y58 peut faire un angle avec le plan de plaque P10 compris entre 0 et 15 degrés et le connecteur peut pivoter autour de l'axe d'extension X42 selon un angle compris entre 0 et 15 degrés. Le connecteur 43 est ainsi un connecteur polyaxial. Les positions du connecteur 43 correspondant à l'angle maximal entre l'axe de connecteur X43 et l'axe d'extension X42 sont représentés en pointillés sur l'insert a) de la figure 8. Les positions du connecteur 43 et de la tige vertébrale 2 correspondant à l'angle maximal entre l'axe de bague Y58 et le plan de plaque P10 sont représentés en pointillés sur l'insert b) de la figure 8.

Le dispositif de connexion 15 est conçu pour évoluer entre une configuration libre et une configuration serrée. En configuration libre, représenté sur l'insert a) de la figure 7 et sur les inserts a) et b) de la figure 8, le connecteur est enfilé sur l'extension 42, une fente 68 sépare les surfaces d'appui 62 et 63 et fend également la bague 58. La tige vertébrale 2 peut alors être facilement introduite dans la bague 58. Le connecteur 43 est libre d'évoluer entre les différentes orientations précédemment décrites.

L'écrou 44 comprend une partie de serrage 70 taraudée, afin de se visser sur l'extension 42, et une surface distale 71 de forme complémentaire à la surface proximale 60. En configuration serrée, représentée sur l'insert b) de la figure 7, l'écrou 44 est vissé sur l'extension 42 et exerce un effort de compression sur le connecteur 43. La surface distale 71 de l'écrou 44 vient alors en appui contre la surface proximale 60 de la calotte proximale 56 selon un contact sphère-sphère. La surface distale 61 de la calotte distale 57 est en appui contre la dépression 30 également selon un contact sphère-sphère et les surfaces d'appui 62 et 63 sont en appui l'une contre l'autre selon l'axe de connecteur X43. La fente 68 a une hauteur mesurée selon l'axe de connecteur X43 nulle. Les calottes proximale 56 et distale 57 sont alors co-radiales, c'est-à-dire que les surfaces proximale 60 et distale 61 forment partie d'une même sphère. La bague 58 est serrée autour de la tige vertébrale 2, de telle sorte que la bague 58 ne peut glisser le long de la tige vertébrale 2. Ainsi, en configuration serrée, les contacts sphère-sphère entre, d'une part l'écrou 44 et la surface proximale 60, et d'autre part la dépression 30 et la surface distale 61 autorisent le connecteur 43 à garder une orientation parmi celles précédemment décrites, sans que le connecteur ne puisse bouger et changer d'orientation, par exemple à cause d'un jeu mécanique. Ainsi, l'écrou 44 fixe l'orientation du connecteur 43, mais ne la définit pas.

La polyaxialité du connecteur 43 facilite le positionnement de la bague 58 par rapport à la tige vertébrale 2, afin que la tige vertébrale 2 ne soit pas contrainte par le positionnement du connecteur 43, mais plutôt que le connecteur 43 soit apte à s'adapter à la position et à l'orientation de la tige vertébrale 2. Cela permet notamment de limiter les contraintes exercées sur la tige vertébrale 2 et sur le système 1 une fois le dispositif de connexion 15 en configuration serrée.

Avantageusement, l'écrou 44 comprend une partie de vissage non représentée, portée par la partie de serrage 70 et conçue pour se détacher de la partie de serrage 70 lorsqu'un couple égal à un couple maximum de vissage est appliqué à la partie de vissage. Ainsi, le couple de vissage appliqué à l'écrou 44 est constant, et l'effort de compression appliqué sur le connecteur 43 est constant. Cela permet notamment à une personne chargée de réaliser le serrage de s'assurer que l'écrou 44 est correctement vissé, et que le connecteur 43 est bien en configuration serrée, limitant ainsi les risques de variations du couple de vissage d'un système 1 à l'autre. En particulier, lorsque le système 1 est en configuration assemblée, le dispositif de connexion 15 est en configuration serrée et l'écrou 44 est vissé sur l'extension 42, au couple égal au couple maximum de vissage.

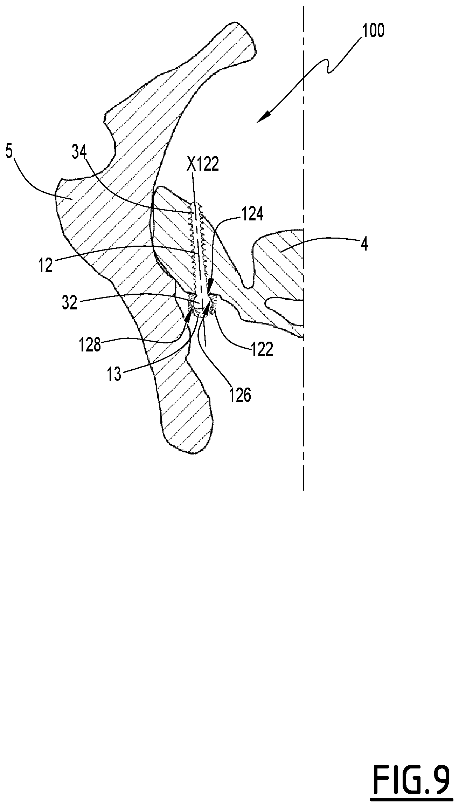

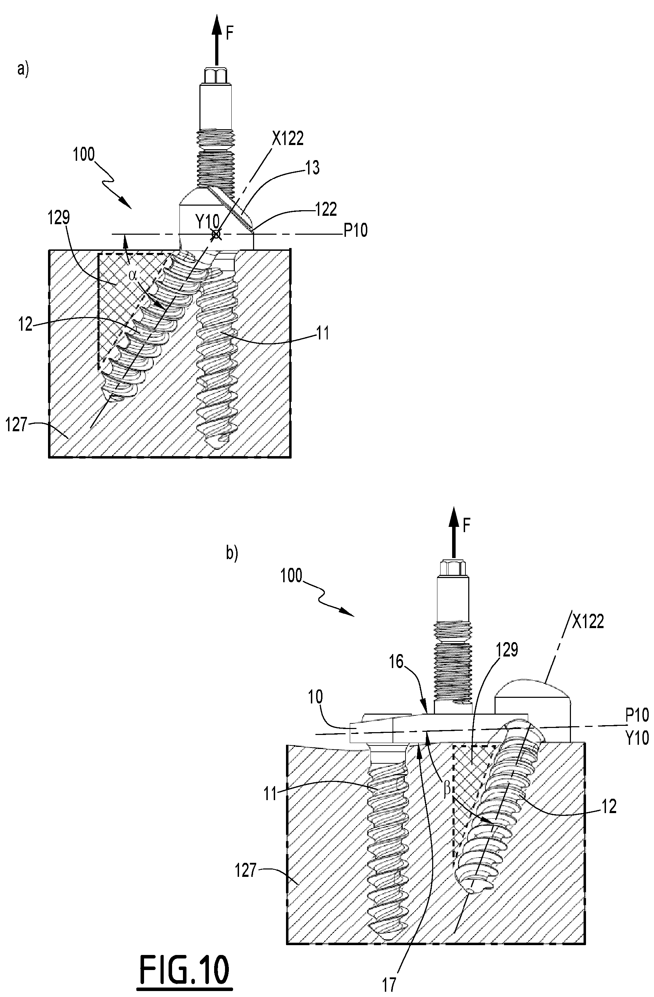

Sur la figure 9 et les inserts a) et b) de la figure 10 est représenté un système 100 en tant que forme de réalisation alternative du système 1. Les éléments du système 100 identiques au système 1 sont désignés par les mêmes signes de référence et ne sont pas décrits à nouveau. Le connecteur 43 et l'écrou 44 ne sont pas représentés.

Le système 100 diffère du système 1 par son deuxième logement 122 qui remplace le deuxième logement 22. Le deuxième logement 122 s'étend selon un deuxième axe X122. Le deuxième axe X122 et l'axe de plaque Y10 forment un deuxième angle β, ce deuxième angle β étant mesuré sur le côté de la plaque 10 destiné à être tourné vers le haut par rapport au patient. Autrement dit, le deuxième angle β est formé dans un plan géométrique, formé par l'axe de plaque Y10 et le deuxième axe X122. Dans la forme de réalisation considérée sur les figures 9 et 10, le deuxième angle β est préférentiellement compris entre 45 et 70 degrés, préférentiellement entre 55 et 65 degrés, voire égal à 60 degrés.

En tenant compte de ce qui précède pour les premier et deuxième modes de réalisation décrits jusqu'ici, on comprend donc que l'invention couvre plus généralement des valeurs du deuxième angle β comprises entre 45 et 110 degrés.

Comme visible sur la figure 9, le deuxième logement 122 comprend un deuxième trou 124, un deuxième fond 126 et une surface taraudée 128. Hormis la valeur du deuxième angle β du système 100, qui diffère de la valeur du deuxième angle β du système 1, le deuxième trou 124, le deuxième fond 126 et le surface taraudée 128 sont fonctionnellement, voire structurellement similaires respectivement aux deuxième trou 24, au deuxième fond 26 et à la surface taraudée 28 du système 1.

Ainsi, lorsque le système 100 est en configuration assemblée, la deuxième vis 12 s'étend en longueur sensiblement selon le deuxième axe X122, c'est-à-dire que son axe longitudinal central se retrouve confondu avec le deuxième axe X122, comme illustré schématiquement sur la figure 9 et les deux inserts a) et b) de la figure 10, ou bien forme avec ce dernier un angle de quelques degrés seulement, typiquement de moins de 15 degrés. Dans tous les cas, l'angulation du deuxième axe X122, telle que définie plus haut au travers des premier et deuxième angles α et β, permet que, en configuration assemblée, la deuxième vis 12 pénètre la vertèbre S2 du sacrum 4, vers le promontoire du sacrum 4, jusqu'à pouvoir, dès lors que la deuxième vis 12 est dimensionnée en longueur de manière ad hoc, traverser la vertèbre S2, sans atteindre l'os iliaque 5. La deuxième vis 12 est avantageusement transfixiante, c'est-à-dire qu'elle traverse la vertèbre S2 de part en part, en particulier les corticales antérieures et postérieures de la vertèbre S2 du sacrum 4 du patient.

De manière similaire à ce qui a été décrit pour le système 1, lorsque le système 100 est en service, il est soumis à la force d'arrachement F.

Pour mieux illustrer ce phénomène, on a représenté sur les inserts a) et b) de la figure 10 les première et deuxième vis 11 et 12 pénétrant dans de la matière osseuse 127, la matière osseuse 127 étant représentée schématiquement par une zone hachurée représentant le sacrum 4 du patient. La matière osseuse 127 comprend une région osseuse 129, représentée en pointillés et doublement hachurée. Cette région osseuse 129 est localisée entre la deuxième vis 12 et le plan de plaque P10 et limite le mouvement de la deuxième vis 12 dû à la force d'arrachement F, ce qui empêche au système 100 d'être arraché. L'existence de la région 129 tient à l'angulation de X122, et notamment de la valeur du premier angle α et, ici, du deuxième angle β. Grâce à l'angulation du deuxième angle β, ici égale à 60 degrés, la région osseuse 129 s'opposant au mouvement de la deuxième vis 12 est de volume supérieur au volume de la région osseuse 29, assurant une bonne tenue mécanique du système 100. Cette meilleure tenue mécanique, assurée par le plus grand volume de la région osseuse 129 comparé à celui de la région osseuse 29, peut compenser partiellement ou totalement une moins bonne tenue mécanique du système 100 due au fait que la deuxième vis 12 ne pénètre pas dans l'os iliaque 5, contrairement à ce qui est prévu pour le système 1.

Le système 100 est avantageux pour des patients pour lesquels il n'est pas possible de pénétrer l'os iliaque 5, assurant une bonne tenue mécanique sans nécessiter de se fixer à la fois à la vertèbre S2 du sacrum 4 du patient et à l'os iliaque 5 du patient. C'est le cas par exemple pour des femmes enceintes, ou qui pourraient tomber enceintes alors qu'elles portent le système 100.

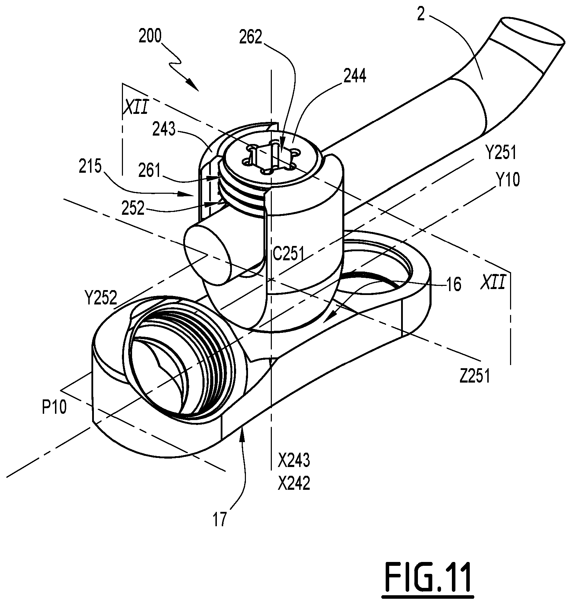

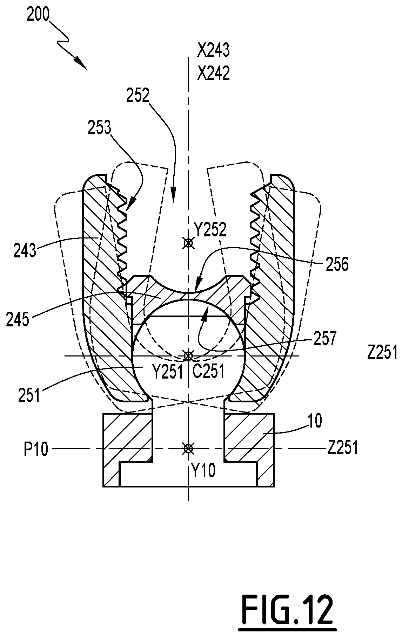

Les figures 11 et 12 représentent un système 200 en tant que forme de réalisation alternative du système 1. Les éléments du système 200 identiques au système 1 sont désignés par les mêmes signes de référence et ne sont pas décrits à nouveau. Les première et deuxième vis 11 et 12 et le bouchon de verrouillage 13 ne sont pas représentés.

Le système 200 diffère du système 1 en ce qu'il comprend un dispositif de connexion 215, qui remplace le dispositif de connexion 15 du système 1. Le dispositif de connexion 15 est situé sur la face proximale 16, entre les deux logements 21 et 22, et aligné sur l'axe de plaque Y10. Le dispositif de connexion 215 comprend un pion 242, une pièce en tulipe 243, s'étendant selon un axe de pièce en tulipe X243, un bouchon 244 et un palet 245. Le bouchon 244 n'est pas représenté sur la figure 12.

Le pion 242, visible à la figure 12, est porté fixement par la plaque 10, tel que l'axe de plaque Y10 passe par le pion 242. Le pion 242 est par exemple soudé à la plaque 10. Le pion 242 s'étend selon un axe de pion X242. Le pion 242 comprend une tête de pion 251, sensiblement sphérique, de centre C251.

La pièce en tulipe 243 s'articule sur la tête de pion 251, plus particulièrement, la pièce en tulipe 243 est en liaison rotule avec la tête de pion 251, centrée sur le centre C251. La pièce en tulipe 243 peut ainsi tourner autour de la tête de pion 251 selon l'axe de pion X242, selon un axe Y251 passant par le centre C251 et parallèle à l'axe de plaque Y10 et selon un axe Z251, perpendiculaire à l'axe de pion X242 et à l'axe de plaque Y10 et passant par le centre C251, mais ne peut pas translater selon ces trois axes X242, Y251 et Z251. Le pivotement maximal de la pièce en tulipe 243 autour de l'axe Y251 est représenté en pointillés sur la figure 12, et l'angle entre l'axe de la pièce en tulipe X243 et l'axe X242 est avantageusement compris entre 0 et 15 degrés. De même, l'angle entre l'axe de la pièce en tulipe X243 et l'axe X242, causé par le pivotement de la pièce en tulipe 243 autour de l'axe Z251 est avantageusement compris entre 0 et 15 degrés. La pièce en tulipe 243 est donc polyaxiale.

La pièce en tulipe 243 comprend une ouverture 252, traversante, s'étendant selon un axe d'ouverture Y252 et comprenant une surface interne 253 taraudée. La tête de pion 251 dépasse dans l'ouverture 252.

Un palet 245 est disposé dans l'ouverture 252, sur la tête de pion 251. Le palet 245 comprend une surface proximale 256 complémentaire à la tige vertébrale 2 et une surface distale 257 complémentaire à la tête de pion 251. Ainsi, lorsque le palet 245 est disposé dans l'ouverture 252, la surface distale 257 du palet 245 est en appui contre la tête de pion 251 et épouse la forme de la tête de pion 251. Le palet 245 est avantageusement monté serré dans l'ouverture 252, de sorte qu'il ne peut pas tomber de l'ouverture 252 et est solidaire de la pièce en tulipe 243. Notamment, il est adapté à pivoter autour de la tête de pion 251 en même temps que la pièce en tulipe 243. La surface proximale 256 étant de forme complémentaire à la tige vertébrale 2, la surface proximale 256 est adaptée pour que la tige vertébrale 2 soit en contact avec la surface proximale 256 en épousant la forme de la tige vertébrale 2. En particulier, la tige vertébrale 2 peut coulisser et pivoter le long de la surface proximale 256. Cela facilite un placement optimal de la tige vertébrale 2 dans la pièce en tulipe 243 lors d'un assemblage du système 200.

Le bouchon 244 comprend une surface extérieure 261 filetée, de sorte à pouvoir se visser dans l'ouverture 252. Une empreinte 262, ménagée dans le bouchon 244, facilite le vissage du bouchon 244 dans l'ouverture 252.

Le dispositif de connexion 215 peut évoluer entre une configuration libre et une configuration serrée. En configuration libre, visible à la figure 12, la pièce en tulipe 243 est libre de pivoter selon le mouvement rotule autour de la tête de pion 251 et de prendre une orientation quelconque par rapport aux axes X242, Y251 et Z251, précédemment décrites. La tige vertébrale 2 peut coulisser et pivoter dans l'ouverture 252 selon l'axe Y252.

Lorsque le dispositif de connexion 215 est en configuration serrée, comme représenté à la figure 11, le bouchon 244 est vissé dans l'ouverture 252 et appuie sur la tige vertébrale 2 et le palet 245. La tige vertébrale 2 est donc en appui contre la surface proximale 256 du palet 245, et, grâce à la complémentarité entre la tige vertébrale 2 et la surface proximale 256, est maintenue immobile dans l'ouverture 252. La surface distale 257 du palet 245 est également en appui sur la tête de pion 251, et, grâce à la complémentarité des surfaces distale 257 et de la tête de pion 251, formant un contact sphère-sphère, le palet 245 est maintenu immobile par rapport à la tête de pion 251. Ainsi, la pièce en tulipe 243 est maintenue immobile par rapport à la tête de pion 251. Les orientations des axes de pièce en tulipe X243 et d'ouverture Y252 sont ainsi fixées. Sur la figure 11, les axes de pièce en tulipe X243 et de pion X242 sont confondus, et les axes Y251 et Y252 sont parallèles.

La polyaxialité du dispositif de connexion 215, comme pour le dispositif de connexion 15, facilite le positionnement de la pièce en tulipe 243 par rapport à la tige vertébrale 2, afin que la tige vertébrale 2 ne soit pas contrainte par le positionnement de la pièce en tulipe 243, mais plutôt que la pièce en tulipe 243 soit apte à s'adapter à la position et à l'orientation de la tige vertébrale 2. Cela permet notamment de limiter les contraintes exercées sur la tige vertébrale 2 et sur le système 200 une fois le dispositif de connexion 215 en configuration serrée.

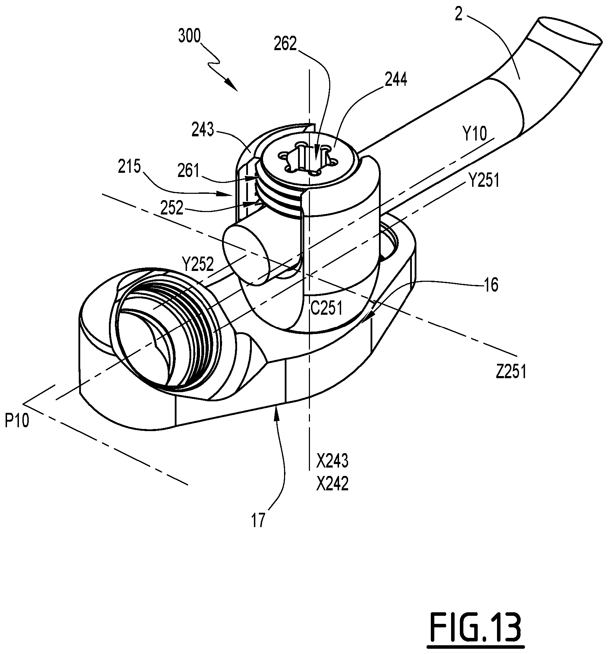

La figure 13 représente un système de fixation sacrée 300, en tant que forme de réalisation alternative du système 200. Les éléments du système 300 identiques au système 200 sont désignés par les mêmes signes de référence et ne sont pas décrits à nouveau. Le système 300 diffère du système 200 en ce que le dispositif de connexion 215 n'est pas aligné sur l'axe de plaque Y10, mais décalé dans une direction perpendiculaire à l'axe de plaque Y10. Cette localisation du dispositif de connexion 215 écarte le dispositif de connexion 215 du deuxième logement 22 et facilite ainsi l'insertion de la tige vertébrale 2 et le vissage de la deuxième vis 12 dans le deuxième logement 22, sans risquer un contact entre la tige vertébrale 2 et la deuxième vis 12 ou le bouchon de verrouillage 13.

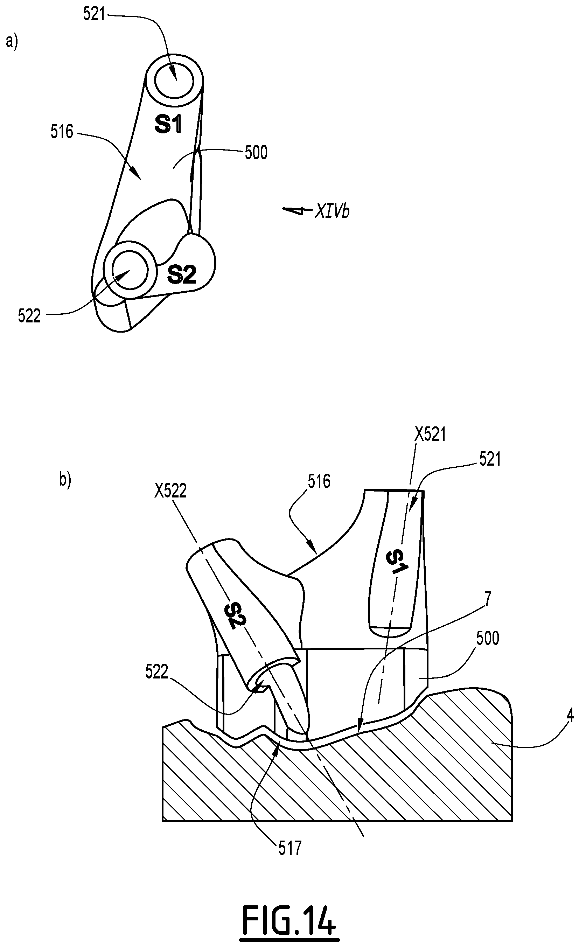

Les inserts a) et b) de la figure 14 représentent un guide de perçage 500, aussi appelé guide 500, appartenant à un ensemble de fixation sacrée comprenant également l'un des systèmes de fixation sacrée 1, 100, 200 ou 300, par exemple le système de fixation sacrée 1. Le guide 500 sert à percer deux pré-trous dans le sacrum 4 du patient, en prévision du vissage de la plaque de fixation 10 sur le sacrum 4 du patient. Les pré-trous percés ont pour objectif de faciliter le vissage des première et deuxième vis 11 et 12, en définissant une orientation des première et deuxième vis 11 et 12 à l'avance.

Le guide de perçage 500 comprend un corps principal 510 comprenant une surface proximale 516 et une surface distale 517, opposée à la surface proximale 516, dans le sens où les faces proximale 516 et distale 517 sont séparées l'une de l'autre par l'épaisseur du corps principal 510, c'est-à-dire la plus petite des trois dimensions du corps principal 510. La surface distale 517 du guide 500 est adaptée à être mise en contact avec le sacrum 4 du patient, en particulier avec une région postérieure 7 du sacrum 4 du patient, et est morpho-adaptée à la région postérieure 7 du patient, c'est-à-dire que la surface distale 517 du guide 500 est complémentaire à la région postérieure 7 du patient. Le guide 500 est adapté pour être placé avec précision afin de recouvrir exactement la région postérieure 7 du patient.

Un premier trou de guide 521 et un deuxième trou de guide 522 relient les surfaces proximale 516 et distale 517 du guide 500 entre elles. Le premier et le deuxième trou de guide 521 et 522 débouchent sur les surfaces proximale et distale 516 et 517 du guide 500, et s'étendent respectivement selon un premier axe de guide X521 et un deuxième axe de guide X522. Une orientation des axes de guide X521 et X522 est identique à l'orientation des première et deuxième vis 11 et 12 lorsque le système 1 est en configuration assemblée.

Lorsque le guide 500 est appliqué sur la région postérieure 7, un utilisateur du guide 500, par exemple un chirurgien, perce un premier pré-trou, percé à travers le premier trou de guide 521, selon l'axe de guide X521. Un deuxième pré-trou, percé à travers le deuxième trou de guide 522 est également percé selon l'axe de guide X522. Ainsi, les pré-trous ont une orientation identique à celle des axes de guide X521 et X522.

Lorsque le système 1 est en configuration assemblée, les première et deuxième vis 11 et 12 sont vissées dans le premier et le deuxième pré-trou respectivement. Ainsi, les orientations des première et deuxième vis 11 et 12 sont respectivement identiques aux orientations des premier et deuxième axe de guide X521 et X522.

## Revendications

1. Système de fixation sacrée (1 ; 100 ; 200 ; 300), comprenant :
- une plaque (10) comprenant :
∘ une face proximale (16) et une face distale (17), opposée à la face proximale (16), un plan de plaque (P10) s'étendant entre les faces distale et proximale,
∘ un premier logement (21) s'étendant selon un premier axe (X21) en reliant entre elles les faces distale (17) et proximale (16), le premier logement (21) incluant à la fois un premier trou (23), qui débouche sur la face distale (17), et un premier fond (25), dans lequel est percé le premier trou (23) et qui relie le premier trou (23) et la face proximale (16),
∘ un deuxième logement (22 ; 122) s'étendant selon un deuxième axe (X22 ; X122) en reliant entre elles les faces distale (17) et proximale (16), le deuxième logement (22 ; 122) incluant à la fois un deuxième trou (24 ; 124), qui débouche sur la face distale (17), une surface taraudée (28 ; 128), qui débouche sur la face proximale (16), et un deuxième fond (26 ; 126), qui est de forme concave, dans lequel est percé le deuxième trou (24 ; 124) et qui relie le deuxième trou (24 ; 124) et la surface taraudée (28 ; 128), et
∘ un axe de plaque (Y10) appartenant au plan de plaque (P10) et sécant avec les premier et deuxième axes (X21, X22 ; X122), le deuxième axe (X22 ; X122) formant, en projection dans un plan perpendiculaire à l'axe de plaque (Y10), un premier angle (α) compris entre 30 et 60 degrés avec le plan de plaque (P10), et le deuxième axe (X22 ; X122) et l'axe de plaque (Y10) formant entre eux un deuxième angle (β) compris entre 45 degrés et 110 degrés ;
- une première vis (11), qui, lorsque le système (1 ; 100 ; 200 ; 300) est dans une configuration assemblée, est reçue dans le premier logement (21), en s'étendant dans le premier trou (23) et en émergeant de la face distale (17) de manière à pouvoir pénétrer dans la vertèbre S1 du sacrum (4) d'un patient ;
- une deuxième vis (12), qui comprend une tige (34) filetée et une tête (32), la tête (32) étant sensiblement sphérique de manière complémentaire au deuxième fond (26 ; 126), la deuxième vis (12) étant, lorsque le système (1 ; 100 ; 200 ; 300) est dans la configuration assemblée, reçue dans le deuxième logement (22 ; 122) de sorte que la tête (32) est en appui sur le deuxième fond (26 ; 126) et la tige (34) s'étend dans le deuxième trou (24 ; 124) et émerge de la face distale (17) de manière à pouvoir pénétrer au moins dans la vertèbre S2 du sacrum (4) du patient ;
- un bouchon de verrouillage (13), qui comprend une jupe (36) dont une face extérieure (37) est filetée de manière complémentaire à la surface taraudée (28 ; 128) et dont une face intérieure (38) est complémentaire de la tête (32) de la deuxième vis (12), et qui, lorsque le système (1 ; 100 ; 200 ; 300) est dans la configuration assemblée, est reçu dans le deuxième logement (22 ; 122) en interposant la jupe (36) radialement entre la tête (32) de la deuxième vis (12) et la surface taraudée (28 ; 128), de manière que la tête (32) de la deuxième vis (12) est reçue dans la jupe (36) et est appuyée contre la face intérieure (38); et
- un dispositif de connexion (15 ; 215), qui, lorsque le système (1 ; 100 ; 200 ; 300) est dans la configuration assemblée, est disposé sur la face proximale (16) entre les premier et deuxième logements (21, 22 ; 122) et relie la plaque (10) à une tige vertébrale (2).

2. Système (1 ; 200 ; 300) selon la revendication 1, dans lequel le deuxième angle (β) est compris entre 70 degrés et 110 degrés, de préférence compris entre 85 et 95 degrés, et la deuxième vis (12) est dimensionnée pour, dans la configuration assemblée, traverser la vertèbre S2 et atteindre l'os iliaque (5) du patient.

3. Système (1 ; 100) selon l'une quelconque des revendications précédentes, dans lequel la plaque (10) comprend en outre une dépression (30) et le dispositif de connexion (15) comprend :
- une extension (42), filetée, liée fixement à la dépression (30) de la plaque (10), l'extension (42) s'étendant selon un axe d'extension (X42);
- un connecteur (43), comprenant une calotte proximale (56) et une calotte distale (57), la calotte distale (57) étant complémentaire de la dépression (30), chaque calotte (56, 57) comprenant :
∘ un orifice (64, 65), de diamètre supérieur à un diamètre de l'extension (42), les orifices (64, 65) respectifs des calottes proximale et distale (56, 57) étant coaxiaux et centrés sur un axe de connecteur (X43),
∘ une surface d'appui (62, 63), les surfaces d'appui (62, 63) étant en regard l'une de l'autre selon l'axe de connecteur (X43);
chaque calotte (56, 57) étant déplaçable selon l'axe de connecteur (X43) afin de mettre les surfaces d'appui (62, 63) en contact l'une avec l'autre selon l'axe de connecteur (X43) et de serrer le connecteur (43) autour de la tige vertébrale (2),
- un écrou (44), comprenant une partie de serrage (70), la partie de serrage (70) comprenant une surface distale (71) complémentaire à la calotte proximale (56),
et dans lequel, lorsque le système (1 ; 100) est en configuration assemblée :
- le connecteur (43) est enfilé sur l'extension (42) par les orifices (64, 65) des calottes distale et proximale (56, 57) ; et
- l'écrou (44) est vissé sur l'extension (42), la surface distale (71) est en appui contre la calotte proximale (56), l'écrou maintient la calotte distale (56, 57) en appui sur la dépression (30) et maintient les surfaces d'appui (62, 63) en contact selon l'axe de connecteur (X43) ; et
- le connecteur (43) est serré autour de la tige vertébrale (2).

4. Système (1 ; 100) selon la revendication 3, dans lequel les calottes proximale et distale (56, 57) sont hémisphériques et lorsque le système (1 ; 100) est en configuration assemblée, les calottes inférieure et supérieure (56, 57) sont co-radiales.

5. Système (1 ; 100) selon l'une quelconque des revendications 3 ou 4, dans lequel l'écrou (44) comprend en outre une partie de vissage, portée par la partie de serrage (70), configurée pour se détacher de la partie de serrage (70) lorsqu'un couple égal à un couple maximum de vissage est appliqué à la partie de vissage.

6. Système (1 ; 100) selon l'une quelconque des revendications 3 à 5, dans lequel l'extension (42) comprend une ligne de moindre résistance (53) la rendant sécable lorsqu'un couple en flexion ou en torsion égal à un couple de rupture en flexion ou en torsion est appliqué à l'extension (42).

7. Système (200 ; 300) selon l'une quelconque des revendications 1 ou 2, dans lequel le dispositif de connexion (215) comprend :
- un pion (242), porté fixement par la plaque (10) ;
- une pièce en tulipe (243) s'étendant selon un axe de pièce en tulipe (X243), le pion (242) et la pièce en tulipe (243) formant entre elles une liaison rotule, la pièce en tulipe (243) comprenant une ouverture (252) traversante taraudée ;
- un palet (245), comprenant une surface distale (257) complémentaire au pion (242) et une surface proximale (256) complémentaire à la tige vertébrale (2) ;
- un bouchon (244), adapté à être vissé dans l'ouverture (252) de la pièce en tulipe (243),
lorsque le système (200 ;300) est dans la configuration assemblée :
- le palet (245) est disposé dans l'ouverture (252) de la pièce en tulipe (243), la surface distale (257) du palet (245) étant en appui contre le pion (242) ;
- la tige vertébrale (2) traverse l'ouverture (252) de la pièce en tulipe (243), en appui contre la surface proximale (256) du palet (245) ;
- le bouchon (244) est vissé dans l'ouverture (252) de la pièce en tulipe (243) et maintient la tige vertébrale (2), le palet (245) et le pion (242) en appui les uns contre les autres.

8. Système (200) selon la revendication 7, dans lequel l'axe de plaque (Y10) passe par le pion (242).

9. Système (300) selon la revendication 7, dans lequel le pion (242) est décalé par rapport à l'axe de plaque (Y10) dans une direction perpendiculaire à l'axe de plaque (Y10).

10. Ensemble de fixation sacrée, comprenant le système (1 ; 100 ; 200 ; 300) selon l'une quelconque des revendications précédentes et un guide de perçage (500) comprenant :
- une surface distale (517) morpho-adaptée à une région postérieure (7) du sacrum (4) du patient ;
- un premier trou de guide (521) centré selon un premier axe de guide (X521), et
- un deuxième trou de guide (522) centré selon un deuxième axe de guide (X522), le premier trou de guide (521) et le deuxième trou de guide (522) étant orientés de manière identique à la première et à la deuxième vis (11, 12) lorsque le système (1 ; 100 ; 200 ; 300) est en configuration assemblée.

## Patentansprüche

1. Sakrales Fixierungssystem (1; 100; 200; 300), umfassend:
- eine Platte (10), umfassend:
o eine proximale Seite (16) und eine distale Seite (17) gegenüberliegt der proximalen Seite (16), wobei sich eine Plattenebene (P10) zwischen der distalen und der proximalen Seite erstreckt,
o eine erste Aufnahme (21), die sich entlang einer ersten Achse (X21) erstreckt, indem sie die distale (17) und die proximale (16) Seite miteinander verbindet, wobei die erste Aufnahme (21) sowohl ein erstes Loch (23), das in die distale Seite (17) mündet, als auch einen ersten Boden (25), in den das erste Loch (23) gebohrt ist und der das erste Loch (23) und die proximale Seite (16) verbindet, beinhaltet,
o eine zweite Aufnahme (22; 122), die sich entlang einer zweiten Achse (X22; X122) erstreckt und die distale (17) und die proximale (16) Seite miteinander verbindet, wobei die zweite Aufnahme (22; 122) sowohl ein zweites Loch (24; 124), das an der distalen Fläche (17) mündet, eine Gewindefläche (28; 128), die an der proximalen Fläche (16) mündet, als auch einen zweiten Boden (26; 126), der konkav geformt ist, beinhaltet, in die das zweite Loch (24; 124) gebohrt ist und die das zweite Loch (24; 124) und die Gewindefläche (28; 128) verbindet, und
o eine Plattenachse (Y10), die zu der Plattenebene (P10) gehört und die erste und zweite Achse (X21, X22; X122) schneidet, wobei die zweite Achse (X22; X122) in Projektion in einer Ebene senkrecht zu der Plattenachse (Y10) einen ersten Winkel (α) bildet, der zwischen 30 und 60 Grad mit der Plattenebene (P10) beträgt, und die zweite Achse (X22; X122) und die Plattenachse (Y10) untereinander einen zweiten Winkel (β) bilden, der zwischen 45 Grad und 110 Grad beträgt;
- eine erste Schraube (11), die, wenn das System (1; 100; 200; 300) in einer zusammengebauten Konfiguration ist, in dem ersten Aufnahme (21) aufgenommen ist, sich in das erste Loch (23) erstreckt und aus der distalen Fläche (17) hervorsteht, sodass sie in den Wirbel S1 des Kreuzbeins (4) eines Patienten eindringen kann;
- eine zweite Schraube (12), die einen Schaft (34) mit Gewinde und einen Kopf (32) umfasst, wobei der Kopf (32) komplementär zu dem zweiten Boden (26; 126) im Wesentlichen kugelförmig ist, wobei die zweite Schraube (12), wenn das System (1; 100; 200; 300) in der zusammengebauten Konfiguration ist, in der zweiten Aufnahme (22; 122) aufgenommen ist, so dass der Kopf (32) auf dem zweiten Boden (26; 126) aufliegt und sich der Schaft (34) in das zweite Loch (24; 124) erstreckt und aus der distalen Fläche (17) hervorsteht, um zumindest in den Wirbel S2 des Kreuzbeins (4) des Patienten einzudringen;
- eine Verschlusskappe (13), die eine Schürze (36) umfasst, deren eine Außenseite (37) komplementär zu der Gewindefläche (28; 128) mit einem Gewinde versehen ist und deren eine Innenseite (38) komplementär zu dem Kopf (32) der zweiten Schraube (12) ist, und die, wenn das System (1; 100; 200; 300) in der zusammengebauten Konfiguration befindet, in der zweiten Aufnahme (22; 122) aufgenommen ist, indem die Schürze (36) radial zwischen dem Kopf (32) der zweiten Schraube (12) und der Gewindefläche (28; 128) angeordnet wird, sodass der Kopf (32) der zweiten Schraube (12) in der Schürze (36) aufgenommen ist und gegen die Innenfläche (38) gedrückt wird; und
- eine Verbindungsvorrichtung (15; 215), die, wenn das System (1; 100; 200; 300) in der zusammengebauten Konfiguration ist, an der proximalen Fläche (16) zwischen der ersten und der zweiten Aufnahme (21, 22; 122) angeordnet ist und die Platte (10) mit einem Wirbelsäulenstab (2) verbindet.

2. System (1; 200; 300) nach Anspruch 1, wobei der zweite Winkel (β) zwischen 70 Grad und 110 Grad, vorzugsweise zwischen 85 und 95 Grad, ist und die zweite Schraube (12) bemessen ist, um in der zusammengebauten Konfiguration den Wirbel S2 zu durchqueren und den Hüftknochen (5) des Patienten zu erreichen.

3. System (1; 100) nach einem der vorherigen Ansprüche, wobei die Platte (10) ferner eine Vertiefung (30) umfasst und die Verbindungsvorrichtung (15) Folgendes umfasst:
- eine Verlängerung (42), die mit einem Gewinde versehen ist und fest mit der Vertiefung (30) der Platte (10) verbunden ist, wobei sich die Verlängerung (42) entlang einer Verlängerungsachse (X42) erstreckt;
- einen Verbinder (43), umfassend eine proximale Kalotte (56) und eine distale Kalotte (57), wobei die distale Kalotte (57) komplementär zu der Vertiefung (30) ist, jede Kalotte (56, 57) umfassend:
o eine Öffnung (64, 65), deren Durchmesser größer ist als ein Durchmesser der Verlängerung (42), wobei die jeweiligen Öffnungen (64, 65) der proximalen und distalen Kalotte (56, 57) koaxial und auf einer Verbinderachse (X43) zentriert sind,
o eine Auflagefläche (62, 63), wobei die Auflageflächen (62, 63) einander entlang der Verbindungsachse (X43) gegenüberliegen;
wobei jede Kalotte (56, 57) entlang der Verbinderachse (X43) verschiebbar ist, um die Auflageflächen (62, 63) entlang der Verbinderachse (X43) miteinander in Kontakt zu bringen und den Verbinder (43) um den Wirbelsäulenstab (2) herum einzuspannen,
- eine Mutter (44), umfassend einen Spannabschnitt (70), wobei der Spannabschnitt (70) eine distale Fläche (71) umfasst, die komplementär zu der proximalen Kalotte (56) ist,
und wobei, wenn das System (1; 100) in der zusammengesetzten Konfiguration ist:
- der Verbinder (43) durch die Öffnungen (64, 65) der distalen und proximalen Kalotte (56, 57) auf die Verlängerung (42) geschoben wird; und
- die Mutter (44) auf die Verlängerung (42) geschraubt wird, die distale Fläche (71) an der proximalen Kalotte (56) anliegt, die Mutter die distale Kalotte (56, 57) an der Vertiefung (30) anliegend hält und die Anlageflächen (62, 63) entlang der Verbinderachse (X43) in Kontakt hält; und
- der Verbinder (43) um den Wirbelsäulenstab (2) festgezogen wird.

4. System (1; 100) nach Anspruch 3, wobei die proximale und die distale Kalotte (56, 57) halbkugelförmig sind und wenn das System (1; 100) in der zusammengebauten Konfiguration ist, die untere und die obere Kalotte (56, 57) koradial sind.

5. System (1; 100) nach einem der Ansprüche 3 oder 4, wobei die Mutter (44) ferner einen Schraubabschnitt umfasst, der von dem Spannabschnitt (70) getragen wird und konfiguriert ist, um sich von dem Spannabschnitt (70) zu lösen, wenn ein Drehmoment, das gleich wie ein maximales Schraubdrehmoment ist, auf den Schraubabschnitt ausgeübt wird.

6. System (1; 100) nach einem der Ansprüche 3 bis 5, wobei die Verlängerung (42) eine Linie des geringsten Widerstands (53) umfasst, die sie trennbar macht, wenn ein Biege- oder Torsionsmoment, das gleich wie ein Biege- oder Torsionsbruchmoment ist, auf die Verlängerung (42) ausgeübt wird.

7. System (200; 300) nach einem der Ansprüche 1 oder 2, wobei die Verbindungsvorrichtung (215) Folgendes umfasst:
- einen Stift (242), der fest von der Platte (10) getragen wird;
- ein Tulpenstück (243), das sich entlang einer Tulpenstückachse (X243) erstreckt, wobei der Stift (242) und das Tulpenstück (243) untereinander eine Kugelgelenkverbindung bilden, wobei das Tulpenstück (243) eine Durchgangsöffnung (252) mit Innengewinde aufweist;
- eine Scheibe (245), umfassend eine zu dem Stift (242) komplementäre distale Fläche (257) und eine zu dem Wirbelsäulenstab (2) komplementäre proximale Fläche (256);
- einen Stopfen (244), der angepasst ist, um in die Öffnung (252) des tulpenförmigen Stücks (243) geschraubt zu werden,
wenn das System (200; 300) in der zusammengesetzten Konfiguration ist:
- ist die Scheibe (245) in der Öffnung (252) des Tulpenstücks (243) angeordnet, wobei die distale Fläche (257) der Scheibe (245) an dem Stift (242) anliegt;
- durchquert der Wirbelsäulenstab (2) die Öffnung (252) des Tulpenstücks (243) und liegt an der proximalen Fläche (256) der Scheibe (245) an;
- ist der Stopfen (244) in die Öffnung (252) des Tulpenstücks (243) geschraubt und hält den Wirbelsäulenstab (2), die Scheibe (245) und den Stift (242) gegeneinander gedrückt.

8. System (200) nach Anspruch 7, wobei die Plattenachse (Y10) durch den Stift (242) verläuft.

9. System (300) nach Anspruch 7, wobei der Stift (242) in Bezug auf die Plattenachse (Y10) in einer Richtung senkrecht zu der Plattenachse (Y10) versetzt ist.

10. Sakrale Fixationsanordnung, umfassend das System (1; 100; 200; 300) nach einem der vorherigen Ansprüche und eine Bohrführung (500), umfassend:
- eine distale Fläche (517), die morphologisch an einen hinteren Bereich (7) des Kreuzbeins (4) des Patienten angepasst ist;
- ein erstes Führungsloch (521), das entlang einer ersten Führungsachse (X521) zentriert ist, und
- ein zweites Führungsloch (522), das entlang einer zweiten Führungsachse (X522) zentriert ist, wobei das erste Führungsloch (521) und das zweite Führungsloch (522) identisch zu der ersten und der zweiten Schraube (11, 12) ausgerichtet sind, wenn das System (1; 100; 200; 300) in der zusammengebauten Konfiguration ist.

## Claims

1. A sacral fixation system (1; 100; 200; 300), comprising:
- a plate (10) comprising:
∘ a proximal face (16) and a distal face (17) opposite the proximal face (16), a plate plane (P10) extending between the distal and proximal faces,
∘ a first housing (21) extending along a first axis (X21) by connecting the distal (17) and proximal (16) faces to each other, the first housing (21) including both a first hole (23), which opens onto the distal face (17), and a first bottom (25), wherein the first hole (23) is drilled, and which connects the first hole (23) and the proximal face (16),
∘ a second housing (22; 122) extending along a second axis (X22; X122) by connecting the distal (17) and proximal (16) faces to each other, the second housing (22; 122) including both a second hole (24; 124), which opens onto the distal face (17), a tapped surface (28; 128), which opens onto the proximal face (16), and a second bottom (26; 126), which has a concave shape, wherein the second hole (24; 124) is drilled, and which connects the second hole (24; 124) and the threaded surface (28; 128), and
∘ a plate axis (Y10) belonging to the plate plane (P10) and intersecting with the first and second axes (X21, X22; X122), the second axis (X22; X122) forming, in projection onto a plane perpendicular to the plate axis (Y10), a first angle (α) comprised between 30 and 60 degrees with the plate plane (P10), and the second axis (X22; X122) and the plate axis (Y10) forming a second angle (β) comprised between 45 degrees and 110 degrees;
- a first screw (11), which, when the system (1; 100; 200; 300) is in an assembled configuration, is received in the first housing (21), extending into the first hole (23) and emerging from the distal face (17) so as to penetrate the vertebra S1 of the sacrum (4) of a patient;
- a second screw (12) which comprises a threaded rod (34) and a head (32), the head (32) being substantially spherical matching the second bottom (26; 126), the second screw (12) being, when the system (1; 100; 200; 300) is in the assembled configuration, received in the second housing (22; 122) such that the head (32) bears against the second bottom (26; 126) and the rod (34) extends into the second hole (24; 124) and emerges from the distal face (17) so as to be able to penetrate at least into the vertebra S2 of the sacrum (4) of the patient;
- a locking cap (13) which comprises a skirt (36) having an outer face (37) threaded to match the tapped surface (28; 128) and an inner face (38) of which matches the head (32) of the second screw (12), and which, when the system (1; 100; 200; 300) is in the assembled configuration, is received in the second housing (22; 122) by interposing the skirt (36) radially between the head (32) of the second screw (12) and the tapped surface (28; 128), so that the head (32) of the second screw (12) is received in the skirt (36) and is pressed against the inner face (38); and
- a connecting device (15; 215) which, when the system (1; 100; 200; 300) is in the assembled configuration, is arranged on the proximal face (16) between the first and second housings (21, 22; 122) and connects the plate (10) to a vertebral rod (2).

2. The system (1; 200; 300) according to claim 1, wherein the second angle (β) is comprised between 70 degrees and 110 degrees, preferably comprised between 85 and 95 degrees, and the second screw (12) is dimensioned, in the assembled configuration, to pass through the vertebra S2 and reach the iliac bone (5) of the patient.

3. The system (1; 100) according to any of the preceding claims, wherein the plate (10) further comprises a recess (30) and the connecting device (15) comprises:
- a threaded extension (42) fixedly connected to the recess (30) of the plate (10), the extension (42) extending along an extension axis (X42);
- a connector (43), comprising a proximal dome cap (56) and a distal dome cap (57), the distal dome cap (57) matching the recess (30), each dome cap (56, 57) comprising:
∘ an orifice (64, 65) with a diameter greater than a diameter of the extension (42), the orifices (64, 65) of the proximal and distal dome caps (56, 57) being coaxial and centered on a connector axis (X43),
∘ a bearing surface (62, 63), the bearing surfaces (62, 63) facing each other along the connector axis (X43);
each dome cap (56, 57) being movable along the connector axis (X43) in order to bring the bearing surfaces (62, 63) into contact with each other along the connector axis (X43) and to tighten the connector (43) around the spinal rod (2),
- a nut (44), including a tightening portion (70), the tightening portion (70) comprising a distal surface (71) matching the proximal dome cap (56),
and wherein, when the system (1; 100) is in the assembled configuration:
- the connector (43) is fitted onto the extension (42) through the ports (64, 65) of the distal and proximal dome caps (56, 57); and
- the nut (44) is screwed onto the extension (42), the distal surface (71) bears against the proximal dome cap (56), the nut holds the distal dome cap (56, 57) against the recess (30) and holds the bearing surfaces (62, 63) in contact along the connector axis (X43); and
- the connector (43) is tightened around the vertebral rod (2).

4. The system (1; 100) according to claim 3, wherein the proximal and distal dome caps (56, 57) are hemispherical and when the system (1; 100) is in the assembled configuration, the lower and upper dome caps (56, 57) are co-radial.

5. The system (1; 100) according to any one of claims 3 or 4, wherein the nut (44) further comprises a screwing portion supported by the tightening portion (70), configured to detach from the tightening portion (70) when a torque equal to a maximum screwing torque is applied to the screwing portion.

6. The system (1; 100) according to any one of claims 3 to 5, wherein the extension (42) includes a line of least resistance (53) making same breakable when a bending or torsional torque equal to a bending or torsional breaking torque is applied to the extension (42).

7. The system (200; 300) according to any of claims 1 or 2, wherein the connection device (215) comprises:
- a pin (242), fixedly supported by the plate (10);
- a tulip-shaped piece (243) extending along a tulip-shaped piece axis (X243), the pin (242) and the tulip-shaped piece (243) forming therebetween, a ball-and-socket linkage, the tulip-shaped piece (243) comprising a threaded through opening (252);
- a disc (245), comprising a distal surface (257) matching the pin (242) and a proximal surface (256) matching the vertebral rod (2);
- a cap (244), suitable for being screwed into the opening (252) of the tulip-shaped piece (243),
when the system (200;300) is in the assembled configuration:
- the disc (245) is arranged in the opening (252) of the tulip-shaped piece (243), the distal surface (257) of the disc (245) bearing against the pin (242);
- the vertebral rod (2) passes through the opening (252) of the tulip-shaped piece (243), bearing against the proximal surface (256) of the disc (245);
- the cap (244) is screwed into the opening (252) of the tulip-shaped piece (243) and holds the vertebral rod (2), the disc (245) and the pin (242) bearing against each other.

8. The system (200) according to claim 7, wherein the plate axis (Y10) passes through the pin (242).

9. The system (300) according to claim 7, wherein the pin (242) is offset from the plate axis (Y10) along a direction perpendicular to the plate axis (Y10).

10. A sacral fixation assembly, comprising the system (1; 100; 200; 300) according to any of the preceding claims and a drill guide (500) comprising:
- a distal surface (517) morpho-adapted to a posterior region (7) of the patient's sacrum (4);
- a first guide hole (521) centered along a first guide axis (X521), and
- a second guide hole (522) centered along a second guide axis (X522), the first guide hole (521) and the second guide hole (522) being oriented identically to the first and the second screws (11, 12) when the system (1; 100; 200; 300) is in the assembled configuration.
